(19) Europäisches Patentamt
European Patent Office
Office européen des brevets

(11) Veröffentlichungsnummer: **0 358 128 B1**

# (12) EUROPÄISCHE PATENTSCHRIFT

(45) Veröffentlichungstag der Patentschrift: **28.06.95**

(51) Int. Cl.[6]: **C07D 207/38**

(21) Anmeldenummer: **89116200.0**

(22) Anmeldetag: **01.09.89**

## (54) Verfahren zur Herstellung von 5-Alkyltetramsäuren.

(30) Priorität: **06.09.88 CH 3337/88**

(43) Veröffentlichungstag der Anmeldung:
**14.03.90 Patentblatt 90/11**

(45) Bekanntmachung des Hinweises auf die Patenterteilung:
**28.06.95 Patentblatt 95/26**

(84) Benannte Vertragsstaaten:
**AT BE CH DE FR GB IT LI NL SE**

(56) Entgegenhaltungen:

**JOURNAL OF THE CHEMICAL SOCIETY, PERKIN TRANSACTIONS I, Band 4, Nr. 6, 1987,Seiten 1177-1182; P. JOUIN et al.: "Stereospecific synthesis of N-protectedstatine and its analogues via chiral tetramic acid"**

**JOURNAL OF THE CHEMICAL SOCIETY, PERKIN TRANSACTIONS I, Band 4, nr. 13, 1972,Seiten 2121-2128; T.P.C. MULHOLLAND et al.: "Synthesis of pyrrolidine-2,4-diones(tetramic acids) and some derivatives"**

(73) Patentinhaber: **LONZA AG
(Geschäftsleitung: 4002 Basel)
CH-3945 Gampel/Wallis (CH)**

(72) Erfinder: **Meul, Thomas, Dr.
10242 Old Orchard Street
La Porte
Texas 77571 (US)**

(74) Vertreter: **Weinhold, Peter, Dr. et al
Patentanwälte
Dr. Weinhold, Dannenberg,
Dr. Gudel, Schubert
Siegfriedstrasse 8
D-80803 München (DE)**

**EP 0 358 128 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF THE CHEMICAL SOCIETY, PERKIN TRANSACTIONS I, Band 4, Nr. 18, 1973, Seiten 2024-2029; G. LOWE et al.: "Synthesis of beta-lactams by photolytic Wolff rearrangement"

TETRAHEDRON LETTERS, Nr. 34, 1978, Seiten 3173-3176, Pergamon Press Ltd, GB; R.C.F. JONES et al.: "A synthesis of 3-acyl-5-alkyl tetramic acids"

CHEMICAL & PHARMACEUTICAL BULLETIN,Band 35, Nr. 10, Oktober 1987, Seiten 4368-4371, Pharmaceutical Society ofJapan; K. HORI et al.: "An efficient 3(C)-acylation of tetramic acids involvingacyl migration of 4(0)-acylates"

JOURNAL OF THE AMERICAN CHEMICAL SOCIETY, Band 96, Nr. 16, 7. August 1974,Seiten 5787-5791; G. STORK et al.: "Carboxy beta-lactams by photochemical ringcontraction"

BULLETIN OF THE CHEMICAL SOCIETY OF JAPAN, Band 49, Nr. 11, November 1976,Seiten 3287-3290; T. KATSUKI et al.: "The stereoselective synthesis of threo-3-hydroxy-4-amino acids"

TETRAHEDRON LETTERS, Band 24, Nr. 43, 1983, Seiten 4755-4756, Pergamon Press, Ltd, GB; R.C.F. JONES et al.: "An unusual bond migration during O-acylation of tetramic acids"

JOURNAL OF ORGANIC CHEMISTRY, Band 47, Nr. 7, 26. März 1982, Seiten 1534-1546,American Chemical Society; E. VEDEJS et al.: "Synthesis of the cytochalasin Disoidolone unit: solutions to the problem of regiochemistry in N-benzoylpyrrolinone Diels-Alder reactions"

PATENT ABSTRACTS OF JAPAN, Band 13, Nr. 236 (C-602), 30. Mai 1989;

HETEROCYCLES, Band 13 (spec. issue), 1979, Seiten 477-495; S. NAKAGAWA et al.:"Structures of BU-2313 A and B, new anti-anaerobic antibiotics and synthesis oftheir analogs"

HETEROCYCLES, Band 26, Nr. 10, 1987, Seiten 2611-2614; M. SATO et al.: "A novelsynthetic method of lactams from 1,3-dioxin-4-ones via intramolecular ketonetrapping"

SYNTHETIC COMMUNICATIONS, Band 19, 1989, Seiten 2573-1583, Marcel Dekker, Inc.; S. KLUTCHKO et al.: "Stereospecific synthesis of S,S-statine and its congeners"

PHYTOCHEMISTRY, Band 27, Nr. 1, 1988, Seiten 77-88, Pergamon Journals Ltd, GB;M.H. LEBRUN et al.: "Relationships between the structure and the phytotoxicityof the fungal toxin tenuazonic acid"

JOURNAL OF MEDICINAL CHEMISTRY, Band 8, Nr. 4, Juli 1965, Seiten 478-482; S.A.HARRIS et al.: "The synthesis of tenuazonic and congeneric tetramic acids"

J.C.S. CHEM. COMM., 1973, Seiten 328-329; G. LOWE et al.: "Synthesis of beta-lactams by photolytic Wolff rearrangement"

THE JOURNAL OF ANTIBIOTICS, Band 33, Nr. 2, Seiten 173-181; S. TODA et al.:"Bu-2313, a new antibiotic complex active against anaerobes. III. Semi-synthesis of Bu-2313 A and B, and their analogs"

## Beschreibung

Die vorliegende Erfindung betrifft ein mehrstufiges Verfahren zur Herstellung von 5-Alkyltetramsäuren aus 4-Alkyloxy- oder 4-Benzyloxy-3-pyrrolin-2-onen und Aldehyden oder Ketonen.

5-Alkyltetramsäuren sind wertvolle Zwischenprodukte für die Herstellung von $\beta$-Hydroxy-$\gamma$-aminosäuren, wie z.B. Statin, das seinerseits eine wesentliche Rolle als Baustein von Renin-Inhibitoren wie z.B. Pepstatin spielt, oder dessen in der Seitenkette modifizierten Analogen. Die Renin-Inhibitoren zeigen vielversprechende physiologische Wirkungen und eignen sich daher zu therapeutischen Zwecken, vor allem als Antihypertensiva (H.J.Altenbach, Nachr.Chem.Tech.Lab. 36, 756 (1988)). Die Tetramsäuren können je nach den Bedingungen und den Substituenten in der Dion-Form, nämlich als Pyrrolidin-2,4-dion oder in der Enolon-Form, nämlich als 4-Hydroxy-3-pyrrolin-2-on oder als Gemisch der beiden Formen vorliegen. Im folgenden ist ungeachtet der tatsächlichen Verhältnisse jeweils nur die Dion-Form abgebildet.
Bisher hat es an einfachen und kostengünstigen Verfahren zur Herstellung verschiedenartig substituierter 5-Alkyl-tetramsäuren gemangelt.
So ist aus Jouin et al., J.Chem.Soc.Perkin Trans.I 1987, 1177 bekannt, N-geschützte $\alpha$-Aminosäuren nach Aktivierung mit Chlorameisensäureisopropenylester in Gegenwart von 4-Dimethylaminopyridin mit Meldrumsäure zu den entsprechenden (1-Hydroxyalkyliden)-meldrumsäuren zu kondensieren, welche beim Erhitzen in Lösung Aceton und $CO_2$ eliminieren und in die N-geschützten 5-substituierten Tetramsäuren übergehen. Dieses Verfahren liefert zwar optisch aktive Tetramsäurederivate, wenn man von den optisch aktiven natürlichen $\alpha$-Aminosäuren ausgeht, es verwendet jedoch eine ganze Reihe von teuren, teilweise schwer zugänglichen oder hochgiftigen Ausgangsmaterialien, was eine technische Anwendung praktisch ausschliesst.
Ein weiterer Nachteil dieses Verfahrens ist die Einschränkung der Variationsmöglichkeiten der Substituenten im Endprodukt, die sich daraus ergibt, dass mit den $\alpha$-Aminosäuren nur eine begrenzte Auswahl an Substituenten zur Verfügung steht.
Die gleichen Nachteile weist ein älteres Verfahren auf, welches von $\alpha$-Aminosäureestern ausgeht, die mit Malonsäureesterchloriden zunächst zu den entsprechenden N-(Alkoxycarbonylacetyl)-$\alpha$-aminosäureestern umgesetzt werden. Diese werden zu den 3-Alkoxycarbonyltetramsäuren cyclisiert, welche durch Hydrolyse und Decarboxylierung in die entsprechenden 5-substituierten Tetramsäuren übergeführt werden. (T.P.C.Mulholland, R.Foster und D. B.Haydock, J.Chem.Soc.Parkin Trans.I 1972, 2121.)

Aufgabe der Erfindung war es daher, ein Verfahren zu finden, das die genannten Nachteile nicht aufweist und ein breites Spektrum verschieden substituierter Tetramsäuren zur Verfügung stellt.
Erfindungsgemäss wird die Aufgabe gemäss Patentanspruch 1 gelöst.

In einem ersten Schritt setzt man ein 3-Pyrrolin-2-on der allgemeinen Formel

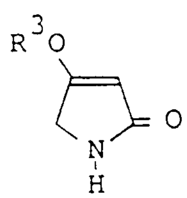

II

worin $R^3$ eine geradkettige oder verzweigte Alkylgruppe mit 1 bis 4 C-Atomen oder eine gegebenenfalls mit einer oder mehreren niedrigen Alkylgruppen substituierte Benzylgruppe ist, mit einem Aldehyd oder Keton der allgemeinen Formel

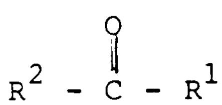

IIIa

oder

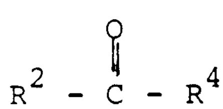

IIIb

worin $R^1$ eine geradkettige oder verzweigte Alkylgruppe mit 1 bis 6 C-Atomen oder eine Cycloalkylgruppe mit 4 bis 7 C-Atomen oder eine Gruppe der Form $-[CH_2]_n-Q$ worin n = 1 oder 2, wobei Q eine der genannten Cycloalkylgruppen oder eine Phenylgruppe ist,
und $R^2$ unabhängig davon Wasserstoff oder eine geradkettige Alkylgruppe mit 1 bis 4 C-Atomen ist
und $R^4$ eine Gruppe ist, die sich von $R^1$ nur durch das Vorhandensein einer oder mehrerer keinem aromatischen System angehörender und nicht zur Carbonylgruppe konjugierter Doppel- oder Dreifachbindungen unterscheidet, um. Die Umsetzung erfolgt in Lösung in Gegenwart einer Base und führt zunächst zu einem 5-Alkyliden-3-pyrrolin-2-on der allgemeinen Formel

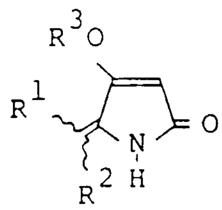

IVa

oder

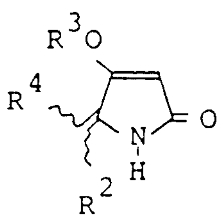

IVb

worin $R^1$, $R^2$, $R^3$ und $R^4$ die oben genannten Bedeutungen haben.
Die 4-Alkoxy- oder 4-Benzyloxy-3-pyrrolin-2-one der allgemeinen Formel II sind nach bekannten Verfahren erhältlich. 4-Alkoxy-3-pyrrolin-2-one können gemäss EP-A 0 216 324 aus 4-Halogenacetessigestern mit Orthoameisensäureestern und Ammoniak hergestellt werden. 4-Benzyloxy-3-pyrrolin-2-one können gemäss EP-A 0 252 363 aus 4-Methoxy-3-pyrrolin-2-on und den entsprechenden Benzylalkoholen hergestellt werden. Als Rest $R^3$ enthalten die 3-Pyrrolin-2-one zweckmässig eine Alkylgruppe mit bis zu 4 C-Atomen, also beispielsweise Methyl, Ethyl, Propyl, Isopropyl oder Butyl oder eine Benzylgruppe, die gegebenenfalls mit einer oder mehreren Alkylgruppen mit bis zu 4 C-Atomen substituiert sein kann, wie beispielsweise o-Methylbenzyl, m-Methylbenzyl, p-Methylbenzyl, 2,4-Dimethylbenzyl, 3,5-Dimethylbenzyl, p-Ethylbenzyl, p-Isopropylbenzyl, p-Butylbenzyl oder p-tert-Butylbenzyl. Bevorzugte Reste $R^3$ sind Methyl, Ethyl, Propyl, Isopropyl oder Benzyl, besonders bevorzugt ist Methyl.

Als Aldehyde oder Ketone der allgemeinen Formel IIIa oder IIIb werden zweckmässig gesättigte aliphatische Aldehyde mit 2 bis 7 C-Atomen, und zwar geradkettige, wie Acetaldehyd, Propionaldehyd, Butyraldehyd, Valeraldehyd, Capronaldehyd oder Oenanthaldehyd, oder verzweigte, wie beispielsweise Isobutyraldehyd, Isovaleraldehyd, Pivalaldehyd, Isocapronaldehyd, 2-Methylvaleraldehyd oder 2-Ethylbutyraldehyd, oder gesättigte alicyclische Aldehyde mit 5 bis 8 C-Atomen, wie beispielsweise Cyclobutancarbaldeyhd, Cyclopentancarbaldehyd, Cyclohexancarbaldehyd oder Cycloheptancarbaldehyd, oder Cycloalkylacetaldehyde, wie beispielsweise Cyclohexylacetaldehyd, Cycloalkyl-propionaldehyde, wie beispielsweise 3-Cyclohexylpropionaldehyd, Arylacetaldehyde, wie Phenylacetaldehyd, Arylpropionaldehyde, wie 3-Phenylpropionaldehyd oder aliphatische Ketone, wie Aceton, Ethyl-methylketon, Isopropyl-methylketon, Diethylketon, Isobutyl-methylketon, 2-Heptanon, 3-Heptanon, 4-Heptanon, 2-Octanon oder 5-Nonanon oder alicyclische Ketone, wie Cyclobutanon, Cyclopentanon, Cyclohexanon oder Cycloheptanon eingesetzt.
Es liegt auch im Rahmen der Erfindung, ungesättigte Aldehyde oder Ketone anstelle der entsprechenden gesättigten zu verwenden, beispielsweise 3-Cyclohexencarbaldehyd statt Cyclohexancarbaldehyd. Voraussetzung ist dabei, dass die Mehrfachbindungen nicht in Konjugation zur Carbonylgruppe stehen, da sonst andere Reaktionswege in den Vordergrund treten. In diesen Fällen werden die Doppel- oder Dreifachbindungen bei dem letzten Verfahrensschritt, der katalytischen Hydrierung, ebenfalls hydriert.

Werden Aldehyde oder unsymmetrische Ketone eingesetzt, so können sich zwei geometrische Isomere, nämlich die Z- oder E-Form des entsprechenden 5-Alkyliden-3-pyrrolin-2-ons bilden. Welche der beiden Formen gebildet wird oder ob beide nebeneinander entstehen, hängt von den Resten $R^1$ bzw. $R^4$ und $R^2$ ab. Für den weiteren Verfahrensablauf ist es unwesentlich, ob die Z- oder die E-Form oder ein Gemisch

entsteht.

Die Reaktion des 3-Pyrrolin-2-ons mit dem Aldehyd oder Keton wird mit einer Base als Katalysator in Lösung durchgeführt. Als Base wird vorzugsweise ein Alkalihydroxid, besonders bevorzugt Natriumhydroxid, eingesetzt.

Als Lösungsmittel eignen sich polare protische Lösungsmittel, wie beispielsweise Wasser oder niedrige Alkohole, vorzugsweise wird Wasser allein oder im Gemisch mit einem niedrigen Alkohol eingesetzt. Die Reaktion wird zweckmässig bei einer Temperatur von 20 bis 100 °C durchgeführt, vorzugsweise bei 30 bis 50 °C. Die Reaktionsdauer beträgt zweckmässig 5 min bis 5 h. Das molare Verhältnis von 3-Pyrrolin-2-on II zu Aldehyd oder Keton III beträgt zweckmässig 1:1 bis 1:5, vorzugsweise 1:1 bis 1:1,5.

Im folgenden Schritt wird das 5-Alkyliden-3-pyrrolin-2-on der allgemeinen Formel III durch Abspaltung des Restes $R^3$ unter Säurekatalyse in eine 5-Alkylidentetramsäure der allgemeinen Formel

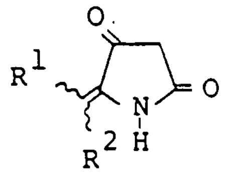

Va

oder

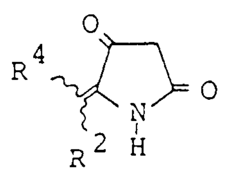

Vb

übergeführt. Dieser Schritt kann übersprungen werden, wenn $R^3$ eine Benzylgruppe oder eine substituierte Benzylgruppe ist, weil Benzylgruppen auch unter den Bedingungen der katalytischen Hydrierung abspaltbar sind (vgl. EP-A 0 252 363). Dies ist besonders dann vorteilhaft, wenn solche erfindungsgemässen Verbindungen hergestellt werden sollen, die unter den Bedingungen der säurekatalysierten Abspaltung zu Nebenreaktionen neigen. Die säurekatalysierte Abspaltung kann mit starken Säuren in polaren protischen Lösungsmitteln, wie Wasser oder wässrigen Lösungsmittelgemischen oder niedrigen Carbonsäuren, durchgeführt werden. In einer bevorzugten Ausführungsform wird Chlor- oder Bromwasserstoff in Essigsäure eingesetzt, besonders bevorzugt ist Chlorwasserstoff. Eine weitere bevorzugte Ausführungsform verwendet Schwefelsäure in wässrigem Tetrahydrofuran oder Dioxan. Die Reaktionstemperatur beträgt zweckmässig 20 bis 100 °C, vorzugsweise 20 bis 60 °C.

Im letzten Verfahrensschritt wird an einem Palladium-Katalysator die exocyclische Doppelbindung sowie gegebenenfalls weitere im Rest $R^4$ vorhandene Doppel- oder Dreifachbindungen hydriert. Gleichzeitig wird, sofern der Rest $R^3$ eine Benzylgruppe oder substituierte Benzylgruppe ist und nicht mittels Säure abgespalten wurde, $R^3$ durch Hydrogenolyse entfernt. Dabei wird in Position 5 des Pyrrolin- bzw. Pyrrolidinringes und, sofern $R^2$ von $R^1$ verschieden und kein Wasserstoff ist, in der $\alpha$-Position der Seitenkette ein Chiralitätszentrum gebildet, so dass die entstehende Tetramsäure als Enantiomeren- bzw. Diastereomerengemisch anfällt.

Der Katalysator kann auf einem Trägermaterial, wie beispielsweise Aktivkohle oder Aluminiumoxid, aufgebracht sein. Die Hydrierung wird zweckmässig in einem Lösungsmittel, wie beispielsweise Methanol oder Essigsäureethylester durchgeführt, es können hierfür alle für katalytische Hydrierungen üblichen Lösungsmittel verwendet werden. Der Wasserstoffdruck bei der Hydrierung ist nicht kritisch, er liegt vorzugsweise bei 1 bis 50 bar. Vorzugsweise wird die Hydrierung bei Temperaturen von 10 bis 60 °C durchgeführt, besonders bevorzugt bei Raumtemperatur.

Die folgenden Beispiele verdeutlichen die Ausführung des erfindungsgemässen Verfahrens.

Beispiele (alle $^1$H-NMR-Spektren wurden in $CDCl_3$ bei 300 MHz aufgenommen)

Beispiel 1
(Z)-4-Methoxy-5-isobutyliden-3-pyrrolin-2-on

(IV, $R^2$ = H, $R^3$ = Me, $R^4$ = Isopropyl)

In 2000 ml 4 n Natronlauge wurden 35,9 g 4-Methoxy-3-pyrrolin-2-on (II, $R^3$ = Me) gelöst und bei 50 °C innerhalb von 30 min mit einer Lösung von 24,0 g Isobutyraldehyd in 675 ml Methanol versetzt. Nach 1 h wurden 675 ml Wasser zugegeben und das Reaktionsgemisch auf 0 °C abgekühlt. Das ausgefallene Produkt wurde abfiltriert, mit Wasser gewaschen und i.Vak. bei 40 °C getrocknet. Das Filtrat wurde mit Dichlormethan extrahiert.

Ausbeute: 39,7 g + 10,1 g aus dem Dichlormethan-Extrakt (99,4% Gesamtausbeute)
Smp.: 139 bis 141 °C, farblose Kristalle
$^1$H-NMR: $\delta$ = 8,64 (br.s, 1H), 5,30 (d, 1H), 5,14 (d, 1H), 3,85 (s, 3H), 2,67 (m, 1H), 1,11 (d, 6H)

Beispiel 2
(Z)-4-Methoxy-5-(cyclohexylmethylen)-3-pyrrolin-2-on

(IV, $R^2$ = H, $R^3$ = Me, $R^4$ = Cyclohexyl)

23,9 g 4-Methoxy-3-pyrrolin-2-on (94,6%ig) in 1360 ml 4 n Natronlauge und 27,5 g Cyclohexancarbaldehyd (90 bis 95%ig) in 330 ml Methanol wurden wie in Beispiel 1 beschrieben umgesetzt.

Ausbeute: 39,8 g (96,1%)
Smp.: 134 bis 136 °C, farblose Kristalle
$^1$H-NMR: $\delta$ = 9,07 (br.s, 1H), 5,32 (d, 1H), 5,14 (d, 1H), 3,83 (s, 3H), 2,40 (m, 1H), 1,09-1,81 (m, 10H)

Beispiel 3
(Z)-4-Methoxy-5-(propyliden)-3-pyrrolin-2-on

(IV, $R^2$ = H, $R^3$ = Me, $R^4$ = Et)

23,9 g 4-Methoxy-3-pyrrolin-2-on (94,6%ig) in 1360 ml 4 n Natronlauge und 13,2 g Propionaldehyd (97%ig) in 330 ml Methanol wurden wie in Beispiel 1 beschrieben umgesetzt.

Ausbeute: 18,0 g (58,8%)
Smp.: 119 bis 127 °C, farblose Kristalle
$^1$H-NMR: $\delta$ = 8,62 (br.s, 1H), 5,43 (t, 1H), 5,12 (d, 1H), 3,84 (s, 3H), 2,27 (m, 2H), 1,12 (t, 3H)

Beispiel 4
(Z)-4-Methoxy-5-(2-ethylbutyliden)-3-pyrrolin-2-on

(IV, $R^2$ = H, $R^3$ = Me, $R^4$ = 3-Pentyl)

Die Synthese erfolgte wie in Beispiel 1 beschrieben, jedoch mit 2-Ethylbutyraldehyd als Carbonylverbindung.

Ausbeute: 73,5%
Smp.: 128 bis 130 °C, farblose Kristalle
$^1$H-NMR: $\delta$ = 8,38 (br.s, 1H), 5,20 (d, 1H), 5,13 (d, 1H), 3,85 (s, 3H), 2,17 (m, 1H), 1,25-1,65 (m, 4H), 0,89 (t, 6H)

Beispiel 5
(±)-(Z)-4-Methoxy-5-(2-methylpentyliden)-3-pyrrolin-2-on

(IV, $R^2$ = H, $R^3$ = Me, $R^4$ = 2-Pentyl)

Die Synthese erfolgte wie in Beispiel 1 beschrieben, jedoch mit 2-Methylvaleraldehyd als Carbonylverbindung.

Ausbeute: 73,3%
Smp.: 83 bis 87 °C, farblose Kristalle
$^1$H-NMR: δ = 8,05 (br.s, 1H), 5,25 (d, 1H), 5,12 (d, 1H), 3,85 (s, 3H), 2,45 (m, 1H), 1,20-1,50 (m, 4H), 1,09 (d, 3H), 0,90 (t, 3H)

Beispiel 6
(Z)-4-Methoxy-5-isopentyliden-3-pyrrolin-2-on

(IV, $R^2$ = H, $R^3$ = Me, $R^4$ = Isobutyl)

Die Synthese erfolgte wie in Beispiel 1 beschrieben, jedoch mit Isovaleraldehyd als Carbonylverbindung.
Ausbeute: 92,8%
Smp.: 90 bis 92 °C, farblose Kristalle
$^1$H-NMR: δ = 8,60 (br.s, 1H), 5,46 (t, 1H), 5,13 (d, 1H), 3,84 (s, 3H), 2,14 (dd, 2H), 1,79 (m, 1H), 0,97 (d, 6H)

Beispiel 7
(Z)-4-Methoxy-5-(2,2-dimethylpropyliden)-3-pyrrolin-2-on

(IV, $R^2$ = H, $R^3$ = Me, $R^4$ = tert-Butyl)

Die Synthese erfolgte wie in Beispiel 1 beschrieben, jedoch mit Pivalaldehyd als Carbonylverbindung.
Ausbeute: 54,5%
Smp.: 165 bis 167 °C, farblose Kristalle
$^1$H-NMR: δ = 6,92 (br.s, 1H), 5,37 (s, 1H), 5,08 (d, 1H), 3,84 (s, 3H), 1,22 (s, 9H)

Beispiel 8
4-Methoxy-5-isopropyliden-3-pyrrolin-2-on

(IV, $R^2$ = $R^3$ = $R^4$ = Me)

Die Synthese erfolgte wie in Beispiel 1 beschrieben, jedoch mit 3 Aequivalenten Aceton als Carbonylverbindung und ohne Zusatz von Methanol.
Ausbeute: 75,8%
Smp.: 246 bis 248 °C, farblose Kristalle
$^1$H-NMR: δ = 8,27 (br.s, 1H), 5,19 (d, 1H), 3,84 (s, 3H), 2,11 (s, 3H), 1,93 (s, 3H)

Beispiel 9
4-Methoxy-5-(1-methylpropyliden)-3-pyrrolin-2-on (E/Z-Gemisch)

(IV, $R^2$ = $R^3$ = Me, $R^4$ = Et)

Die Synthese erfolgte wie in Beispiel 8 beschrieben, jedoch mit 2-Butanon als Carbonylverbindung.
Ausbeute: 34,7%
Smp.: 119 bis 122 °C, farblose Kristalle
$^1$H-NMR: δ = 7,29 (br.s, 1H), 5,18 (d, 1H), 3,82 (s, 3H), 2,52 (q, 1H), 2,25 (q, 1H), 2,08 (s, 3H), 1,95 (s, 3H), 1,11 (t, 3H), 1,07 (t, 3H)

Beispiel 10
(±)-(Z)-4-Methoxy-5-(3-cyclohexen-1-yl-methylen)-3-pyrrolin-2-on

(IV, $R^2$ = H, $R^3$ = Me, $R^4$ = 3-Cyclohexen-1-yl)

Die Synthese erfolgte wie in Beispiel 1 beschrieben, jedoch mit 3-Cyclohexen-1-aldehyd (1,2,3,6-Tetrahydrobenzaldehyd) als Carbonylverbindung.
Ausbeute: 97,1%
Smp.: 152 bis 162 °C, farblose Kristalle

$^1$H-NMR: δ = 7,87 (br.s, 1H), 5,62-5,79 (m, 2H), 5,40 (d, 1H), 5,13 (d, 1H), 3,84 (s, 3H), 2,58 (m, 1H), 1,44-2,29 (m, 6H)

Beispiel 11
(Z)-4-Benzyloxy-5-isobutyliden-3-pyrrolin-2-on

(IV, $R^2$ = H, $R^3$ = Benzyl, $R^4$ = Isopropyl)

Diese Synthese erfolgte wie in Beispiel 1 beschrieben, jedoch mit 4-Benzyloxy-3-pyrrolin-2-on (II, $R^3$ = Benzyl) anstelle von 4-Methoxy-3-pyrrolin-2-on.

Ausbeute: 57,6%
Smp.: 159 bis 161 °C, farblose Kristalle
$^1$H-NMR: δ = 8,17 (br.s, 1H), 7,30-7,45 (m, 5H), 5,38 (d, 1H), 5,20 (d, 1H), 5,03 (s, 2H), 2,62 (m, 1H), 1,11 (d, 6H)

Beispiel 12
(Z)-5-Isobutylidenpyrrolidin-2,4-dion ((Z)-5-Isobutylidentetramsäure)

(Va, $R^1$ = Isopropyl, $R^2$ = H)

In 390 ml Essigsäure wurden 39,7 g (Z)-4-Methoxy-5-isobutyliden-3-pyrrolin-2-on (hergestellt gemäss Beispiel 1) gelöst. Die Lösung wurde bei 40 bis 45 °C innerhalb von 10 h mit Chlorwasserstoffgas gesättigt und anschliessend i.Vak. eingeengt.

Ausbeute: 49,3 g
Smp.: 140 bis 142 °C (aus Wasser), gelbliche Kristalle
$^1$H-NMR: δ = 9,68 (br.s, 1H), 5,58 (d, 1H), 3,12 (s, 2H), 2,55 (m, 1H), 1,12 (d, 6H)

Beispiele 13 bis 20

Analog zu Beispiel 12 wurden die in Tabelle 1 aufgeführten Verbindungen hergestellt. Die Ausbeuten sind fast quantitativ (>95%), alle Verbindungen sind gelb.

Tabelle 1

| Beispiel | Name | Struktur | Edukt aus Beispiel | Smp. [°C] | $^1$H-NMR-Spektrum |
|---|---|---|---|---|---|
| 13 | (Z)-5-(Cyclohexyl-methylen)-pyrrolidin-2,4-dion | Va, $R^1$ = Cyclohexyl, $R^2$ = H | 2 | 168-170 | 9,37 (br.s, 1H), 5,61 (d, 1H), 3,12 (s, 2H), 2,22 (m, 1H), 1,13-1,82 (m, 10H) |
| 14 | (Z)-5-Propyliden-pyrrolidin-2,4-dion | Va, $R^1$ = Et, $R^2$ = H | 3 | 134-136 | 10,07 (br.s, 1H), 5,72 (t, 1H), 3,13 (s, 2H), 2,20 (m, 2H), 1,13 (t, 3H) |
| 15 | (Z)-5-(2-Ethylbutyliden)-pyrrolidin-2,4-dion | Va, $R^1$ = 3-Pentyl, $R^2$ = H | 4 | 127-129 | 9,78 (br.s, 1H), 5,51 (d, 1H), 3,13 (s, 2H), 2,12 (m, 1H), 1,25-1,68 (m, 4H), 0,89 (t, 6H) |
| 16 | (±)-(Z)-(1-Methyl-pentyliden)-pyrrolidin-2,4-dion | Va, $R^1$ = 2-Pentyl, $R^2$ = H | 5 | 115-117 | 9,40 (br.s, 1H), 5,53 (d, 1H), 3,12 (s, 2H), 2,38 (m, 1H), 1,20-1,53 (m, 4H), 1,09 (d, 3H), 0,91 (t, 3H) |
| 17 | (Z)-5-Isopentyliden-pyrrolidin-2,4-dion | Va, $R^1$ = Isobutyl, $R^2$ = H | 6 | 114-115 | 9,97 (br.s, 1H), 5,76 (t, 1H), 3,12 (s, 2H), 2,09 (dd, 1H), 1,82 (m, 1H), 0,97 (d, 6H) |
| 18 | (Z)-5-(2,2-Dimethyl-propyliden)-pyrrolidin-2,4-dion | Va, $R^1$ = tert-Butyl, $R^2$ = H | 7 | 106-108 | 8,42 (br.s, 1H), 5,67 (s, 1H), 3,04 (s, 2H), 1,22 (s, 9H) |
| 19 | 5-Isopropyliden-pyrrolidin-2,4-dion | Va, $R^1$ = $R^2$ = Me | 8 | 187-188 | 9,43 (br.s, 1H), 3,11 (s, 2H), 2,20 (s, 3H), 1,89 (s, 3H) |
| 20 | (±)-(Z)-5-(3-Cyclohexen-1-yl-methylen)-pyrrolidin-2,4-dion | Vb, $R^2$ = H, $R^4$ = 3-Cyclohexen-1-yl | 10 | | 5,60-5,80 (m, 3H), 3,13 (s, 2H), 2,55 (m, 1H), 1,45-2,30 (m, 6H) |

Beispiel 21
(±)-5-Isobutyl-pyrrolidin-2,4-dion ((±)-5-Isobutyltetramsäure)

(I, $R^1$ = Isopropyl, $R^2$ = H)

In 200 ml Essigsäureethylester wurden 10,0 g (Z)-5-Isobutyliden-pyrrolidin-2,4-dion (Rohprodukt aus Beispiel 12) gelöst und mit 1,0 g Palladium/Aktivkohle (5% Pd) versetzt. Bei Raumtemperatur und 20 bar Wasserstoffdruck wurde im Autoklaven unter Rühren 4 h hydriert, anschliessend der Katalysator abfiltriert und das Lösungsmittel abdestilliert.

| | |
|---|---|
| Ausbeute: | 7,4 g Rohprodukt (98% bezogen auf 5-Isobutyliden-4-methoxy-3-pyrrolin-2-on) |
| Smp.: | 113 bis 117 °C (aus Essigsäureethylester/Hexan), gelbliche Kristalle |
| $^1$H-NMR: | $\delta$ = 8,05 (br.s, 1H), 4,04 (dd, 1H), 3,04 (s, 2H), 1,44-1,89 (m, 3H), 0,97 (dd, 6H) |

Beispiele 22 bis 26

Analog zu Beispiel 21 wurden die in Tabelle 2 aufgeführten Verbindungen hergestellt. Die Ausbeuten beziehen sich jeweils auf die entsprechende Verbindung V, alle Verbindungen sind farblos.

Tabelle 2

| Beispiele | Name | Struktur | Edukt aus Beispiel | Smp. [°C] | Ausbeute [%] | $^1$H-NMR-Spektrum |
|---|---|---|---|---|---|---|
| 22 | (±)-5-(Cyclohexylmethyl)-pyrrolidin-2,4-dion | I, $R^1$ = Cyclohexyl, $R^2$ = H | 13 | 169-171 | 83,2 | 7,00 (br.s, 1H), 4,07 (dd, 1H), 3,04 (s, 2H), 0,85-1,80 (m, 13H) |
| 23 | (±)-5-(2-Ethylbutyl)-pyrrolidin-2,4-dion | I, $R^1$ = 3-Pentyl, $R^2$ = H | 15 | 78-80 | 71,7 | 7,30 (br.s, 1H), 4,04 (dd, 1H), 3,03 (s, 2H), 1,22-1,84 (m, 7H), 0,82-0,97 (m, 6H) |
| 24 | (±)-5-Propyl-pyrrolidin-2,4-dion | I, $R^1$ = Et, $R^2$ = H | 14 | 101-103 | 97,6 | 7,20 (br.s, 1H), 4,03 (dd, 1H), 3,03 (s, 2H), 1,32-1,90 (m, 4H), 0,98 (t, 3H) |
| 25 | (±)-5-Isopentyl-pyrrolidin-2,4-dion | I, $R^1$ = Isobutyl, $R^2$ = H | 17 | 124-126 | 88 | 7,09 (br.s, 1H), 4,01 (dd, 1H), 3,02 (s, 2H), 1,12-1,91 (m, 5H), 0,92 (dd, 6H) |
| 26 | (±)-5-(2-Methylpentyl)-pyrrolidin-2,4-dion (Diastereomerengemisch) | I, $R^1$ = 2-Pentyl, $R^2$ = H | 16 | 98-101 | 73 | 7,10 (br.s, 1H)* 6,98 (br.s, 1H)** 4,00-4,10 (m, 1H)*** 3,03 (s,2H)*** 0,85-1,90 (m,13H)*** |

* : Diastereomer A
** : Diastereomer B
***: Diastereomere A+B

Beispiel 27
(±)-5-Isobutyl-pyrrolidin-2,4-dion

(I, $R^1$ = Isopropyl, $R^2$ = H)

In 50 ml Essigsäureethylester wurden 4,0 g (Z)-4-Benzyloxy-5-isobutyliden-3-pyrrolin-2-on (hergestellt gemäss Beispiel 11) gelöst und mit 0,4 g Palladium/Aktivkohle (5% Pd) versetzt. Bei Raumtemperatur und 20 bar Wasserstoffdruck wurde im Autoklaven under Rühren 7 h hydriert, anschliessend der Katalysator abfiltriert und das Lösungsmittel abdestilliert.
Ausbeute: 2,6 g, farblose Kristalle
Physikalische Daten identisch mit dem Produkt gemäss Beispiel 21.

**Patentansprüche**

1. Verfahren zur Herstellung von substituierten Tetramsäuren der allgemeinen Formel

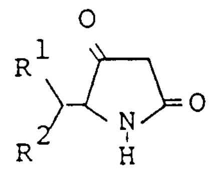

I

oder ihrer Tautomeren, worin

a) $R^1$ eine geradkettige oder verzweigte Alkylgruppe mit 1 bis 6 C-Atomen oder eine Cycloalkylgruppe mit 4 bis 7 C-Atomen, oder eine Gruppe der Form $-[CH_2]_n-Q$, worin n = 1 oder 2 und Q eine der genannten Cycloalkylgruppen oder eine Phenylgruppe ist, und $R^2$ unabhängig davon Wasserstoff oder eine geradkettige Alkylgruppe mit 1 bis 4 C-Atomen ist oder

b) $R^1$ und $R^2$ zusammen eine gegebenenfalls verzweigte Alkandiylgruppe, die in Verbindung mit dem verknüpfenden C-Atom einen gegebenenfalls mit einer oder mehreren niedrigen Alkylgruppen substituierten 4- bis 7-gliedrigen Ring bildet, sind,

dadurch gekennzeichnet, dass man ein 3-Pyrrolin-2-on der allgemeinen Formel

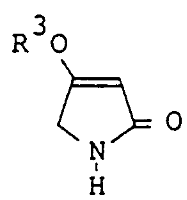

II

worin $R^3$ eine geradkettige oder verzweigte Alkylgruppe mit 1 bis 4 C-Atomen, z.B. Methyl, oder eine gegebenenfalls mit einer oder mehreren niedrigen Alkylgruppen substituierte Benzylgruppe ist, mit einem Aldehyd oder Keton der allgemeinen Formel

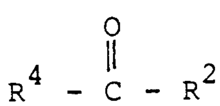

III

in der entweder $R^2$ und $R^4$ die oben für $R^2$ und $R^1$ genannten Bedeutungen haben oder $R^4$ bzw. die durch $R^2$ und $R^4$ zusammen gebildete gegebenenfalls substituierte Alkandiylgruppe sich von $R^1$ bzw. der von $R^1$ und $R^2$ zusammen gebildeten Alkandiylgruppe durch das Vorhandensein einer oder mehrerer keinem aromatischen System angehörender und zur Carbonylgruppe nicht konjugierter Doppel- oder Dreifachbindungen unterscheidet, in Gegenwart einer Base in Lösung zu einem 5-Alkyliden-3-pyrrolin-2-on der allgemeinen Formel

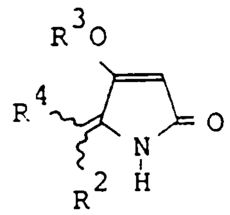

IV

in der $R^2$, $R^3$ und $R^4$ die oben genannten Bedeutungen haben, umsetzt, von diesem den Rest $R^3$ abspaltet und die exocyclische Doppelbindung sowie gegebenenfalls im Rest $R^4$ vorhandene Mehrfachbindungen katalytisch hydriert.

**2.** Verfahren nach Patentanspruch 1, dadurch gekennzeichnet, dass die Abspaltung des Restes $R^3$ durch Behandlung mit einer starken Säure erfolgt und die katalytische Hydrierung anschliessend vorgenommen wird, wobei diese Abspaltung z.B. mit einer Säure aus der Gruppe Chlorwasserstoff, Bromwasserstoff oder Schwefelsäure in einem Lösungsmittel der Gruppe Wasser, Essigsäure, wässriges Tetrahydrofuran, wässriges Dioxan oder einem Gemisch dieser Lösungsmittel durchgeführt wird.

**3.** Verfahren nach Patentanspruch 1, dadurch gekennzeichnet, dass $R^3$ ein gegebenenfalls substituierter Benzylrest ist und die Abspaltung von $R^3$ gemeinsam mit der katalytischen Hydrierung der exocyclischen Doppelbindung und gegebenenfalls in Rest $R^4$ vorhandener Mehrfachbindungen in einem Schritt an einem Palladium-Katalysator erfolgt.

**4.** Verfahren nach mindestens einem der Patentansprüche 1 bis 3, dadurch gekennzeichnet, dass die Kondensation des 3-Pyrrolin-2-ons mit der Carbonylverbindung in wässriger oder wässrig-alkoholischer Lösung bei 20 bis 100 °C durchgeführt wird.

**5.** Verfahren nach mindestens einem der Patentansprüche 1 bis 4, dadurch gekennzeichnet, dass als Base ein Alkalihydroxid eingesetzt wird.

**6.** Verfahren nach Patentansprüchen 4 bzw. 5, dadurch gekennzeichnet, dass die Kondensation des 3-Pyrrolin-2-ons mit der Carbonylverbindung bei 20 bis 50 °C durchgeführt wird.

**7.** Verfahren nach Patentanspruch 2, dadurch gekennzeichnet, dass die Abspaltung der Gruppe $R^3$ mit Chlorwasserstoff in wasserfreier Essigsäure bei einer Temperatur von 20 bis 60 °C durchgeführt wird.

**8.** Verfahren nach mindestens einem der Patentansprüche 1 bis 7, dadurch gekennzeichnet, dass als Katalysator Palladium auf Aktivkohle verwendet wird.

**9.** Verfahren nach mindestens einem der Patentansprüche 1 bis 8, dadurch gekennzeichnet, dass die Hydrierung in einem gegen katalytische Hydrierung inerten Lösungsmittel unter einem Druck von 1 bis 50 bar durchgeführt wird.

## Claims

**1.** Process for the preparation of substituted tetramic acids of general formula

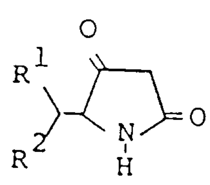

I

or of tautomers thereof, wherein

a) $R^1$ is a straight or branched alkyl group having 1 to 6 carbon atoms or a cycloalkyl group having 4 to 7 C-atoms or a group $-[CH_2]_n-Q$ wherein n = 1 or 2 and Q is one of the mentioned cycloalkyl groups or a phenyl group, and wherein $R^2$ independently is hydrogen or a straight alkyl group having 1 to 4 C-atoms, or

b) $R^1$ and $R^2$ together are an optionally branched alkanediyl group which together with the linking C-atom constitutes a 4- to 7-membered ring, optionally substituted with one or more lower alkyl groups,

characterised in that a 3-pyrroline-2-one of general formula

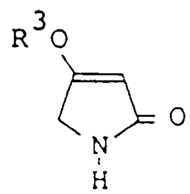

II

wherein $R^3$ is a straight or branched alkyl group having 1 to 4 C-atoms, e.g. methyl, or a benzyl group, optionally substituted with one or more lower alkyl groups, is reacted with an aldehyde or a ketone of general formula

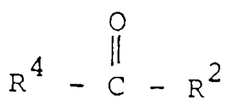

III

wherein either $R^2$ and $R^4$ have the meanings given above for $R^2$ and $R^1$; or $R^4$ and, respectively, the optionally substituted alkanediyl group constituted by $R^2$ and $R^4$ together differ from $R^1$ and, respectively, the alkanediyl group constituted by $R^1$ and $R^2$ together in that one or more double or triple bonds are present which do not belong to an aromatic system and are not conjugated with the carbonyl group, in the presence of a base in solution to give a 5-alkylidene-3-pyrroline-2-one of general formula

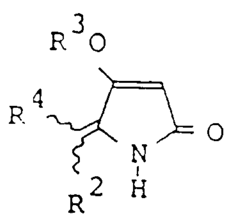

IV

wherein $R^2$, $R^3$ and $R^4$ have the above meanings, the residue $R^3$ is cleaved off and the exocyclic double bond and the multiple bonds optionally present in the residue $R^4$ are catalytically hydrogenated.

**2.** Process according to claim 1, characterised in that the cleavage of residue $R^3$ is accomplished by treatment with a strong acid and that the catalytic hydrogenation is carried out subsequently, said cleavage being carried out e.g. with an acid from the group of hydrochloric acid, hydrobromic acid or sulfuric acid in a solvent of the group of water, acetic acid, aqueous tetrahydrofurane, aqueous dioxane or a mixture of said solvents.

**3.** Process according to claim 1, characterised in that $R^3$ is an optionally substituted benzyl residue and that the cleavage of $R^3$ is carried out simultaneously in a single step with the catalytic hydrogenation of the exocyclic double bond and the multiple bonds optionally present in residue $R^4$ using a palladium catalyst.

**4.** Process according to at least one of claims 1 to 3, characterised in that the condensation of the 3-pyrroline-2-one with the carbonyl compound is carried out in aqueous or aqueous-alcoholic solution at 20 to 100 °C.

5. Process accoring to at least one of claims 1 to 4, characterised in that an alkali hydroxide is used as the base.

6. Process according to claim 4 and 5, respectively, characterised in that the condensation of the 3-pyrroline-2-one with the carbonyl compound is carried out at 20 to 50 °C.

7. Process according to claim 2, characterised in that the cleavage of the group $R^3$ is carried out with hydrogen chloride in anhydrous acetic acid at a temperature of from 20 to 60 °C.

8. Process according to at least one of claims 1 to 7, characterised in that palladium on carbon is used as the catalyst.

9. Process according to at least one of claims 1 to 8, characterised in that the hyrogenation is carried out in a solvent inert towards catalytic hydrogenation at a pressure of from 1 to 50 bar.

**Revendications**

1. Procédé pour la préparation d'acides tétramiques substitués de la formule générale

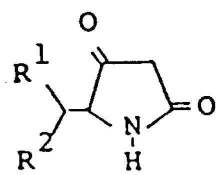

I

ou de leurs tautomères, dans laquelle

a) $R^1$ est un groupe alkyle linéaire ou ramifié avec 1 jusqu'à 6 atomes C ou un groupe cycloalkyle avec 4 jusqu'à 7 atomes C, ou un groupe de la forme $-[CH_2]_n-Q$, dans laquelle n = 1 ou 2 et Q est l'un des groupes cycloalkyle précités ou un groupe phényle, et $R^2$ indépendamment de ce qui précède, est l'hydrogène ou un groupe alkyle linéaire avec 1 jusqu'à 4 atomes C, ou

b) $R^1$ et $R^2$ sont conjointement un groupe alcandiyle éventuellement ramifié, qui avec l'atome C lié, forme un cycle à 4 jusqu'à 7 éléments éventuellement substitués par un ou plusieurs groupes alkyle inférieurs,

caractérisé en ce que l'on met en réaction une 3-pyrrolin-2-one de la formule générale

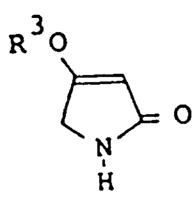

II

dans laquelle $R^3$ est un groupe alkyle linéaire ou ramifié avec 1 jusqu'à 4 atomes C par exemple méthyle ou un groupe benzyle, éventuellement substitué par un ou plusieurs groupes alkyle inférieurs, avec un aldéhyde ou une cétone de la formule générale

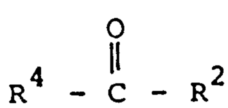

III

dans laquelle $R^2$ et $R^4$ qui ont les significations précitées pour $R^2$ et $R^1$ ou $R^4$ ou le groupe alcandiyle éventuellement substitué par $R^2$ et $R^4$ formés conjointement se différencie de $R^1$ ou du groupe alcandiyle formé conjointement par $R^1$ et $R^2$ par la présence d'une ou plusieurs liaisons double ou triple

non conjuguées au groupe carbonyle et ne faisant pas partie d'un système aromatique, en présence d'une base dans une solution en une 5-alkyliden-3-pyrrolin-2-one de la formule générale

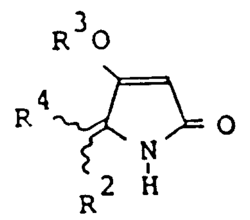

IV

dans laquelle $R^2$, $R^3$ et $R^4$ ont les significations précitées, on sépare de celles-ci le radical $R^3$ et l'on procède à l'hydrogénation catalytiquement de la double liaison exocyclique ainsi qu'éventuellement des liaisons multiples présentes dans le radical $R^4$.

**2.** Procédé selon la revendication 1, caractérisé en ce que la séparation du radical $R^3$ s'effectue par traitement avec un acide fort et l'hydrogénation catalytique est effectuée consécutivement, la séparation par exemple avec un acide du groupe chlorhydrique, bromhydrique ou acide sulfurique dans un solvant du groupe eau, acide acétique, tétrahydrofuranne aqueux, dioxane aqueux ou un mélange de ces solvants.

**3.** Procédé selon la revendication 1, caractérisé en ce que $R^3$ est un reste benzyle éventuellement substitué et la séparation de $R^3$ conjointement avec l'hydrogénation catalytique de la liaison double exocyclique éventuellement dans des liaisons multiples présentes dans le radical $R^4$ s'effectue en une étape sur un catalyseur au palladium.

**4.** Procédé selon au moins l'une des revendications 1 à 3, caractérisé en ce que la condensation de la 3-pyrrolin-2-one s'effectue avec le composé carbonyle dans une solution aqueuse ou aqueuse-alcoolique à 20 jusqu'à 100 °C.

**5.** Procédé selon au moins l'une des revendications 1 à 4, caractérisé en ce qu'en tant que base, on met en oeuvre un hydroxyde alcalin.

**6.** Procédé selon les revendications 4 et 5, caractérisé en ce que la condensation de la 3-pyrrolin-2-one s'effectue avec le composé carbonyle à 20 jusqu'à 50 °C.

**7.** Procédé selon la revendication 2, caractérisé en ce que la séparation du groupe $R^3$ avec le chlorure d'hydrogène s'effectue dans l'acide acétique anhydre à une température de 20 à 60 °C.

**8.** Procédé selon au moins l'une des revendications 1 à 7, caractérisé en ce qu'en tant que catalyseur, on utilise du palladium sur du charbon actif.

**9.** Procédé selon au moins l'une des revendications 1 à 8, caractérisé en ce que l'hydrogénation s'effectue dans un solvant inerte contre hydrogénation catalytique à une pression de 1 à 50 bars.